# EUROPÄISCHE PATENTANMELDUNG

(11) **EP 1 929 931 A2**
(43) Veröffentlichungstag der Anmeldung: **11.06.2008**
(21) Anmeldenummer: 07076034.3
(22) Anmeldetag: 30.11.2007
(51) Int. Cl.: A61B 1/00, G01B 21/00

(54) **Fernsteuerungssystem für medizinische Geräte**

(30) Priorität: 05.12.2006 DE 102006058359
(71) Anmelder: Carl Zeiss Surgical GmbH, 73447 Oberkochen (DE)
(72) Erfinder: Quendt, Dieter, 73457 Esslingen (DE)
(74) Vertreter: Theobald, Andreas

(57) **Zusammenfassung**

Es wird ein kabelloses Fernsteuerungssystem (2) für ein medizinisches Gerät zur Verfügung gestellt. Das Fernsteuerungssystem (2) umfasst einen ersten Akku (16), einen zweiten Akku (18), ein Steuermodul (10) zum Anbringen oder zum Einbauen in das medizinische Gerät (8), wenigstens eine Fernsteuerbedieneinheit (6) und eine Ladestation (4). Der erste Akku (16) ist in der Fernsteuereinheit (6) angeordnet ist, der zweite Akku ist der Ladeeinheit (4) zugeordnet.

## Beschreibung

Die Erfindung betrifft ein kabelloses Fernsteuerungssystem für medizinische Geräte und ein Verfahren zum Aufladen eines Akkus einer Fernsteuerung.

Bei kabellosen Fernsteuerungen für chirurgische Geräte oder optische Beobachtungsgeräte, insbesondere Operationsmikroskope, werden die notwendigen Steuersignale statt über ein Kabel drahtlos übertragen. Dazu wird eine Netzwerkverbindung, beispielsweise Bluetooth, verwendet. Für eine medizinische Fernsteuerung bietet sich Bluetooth als Übertragungstechnik an, weil die verwendete Frequenz weltweit zulässig ist, und die Übertragungstechnik relativ sicher gegen Störungen ist. Zum Aufbau der für das Netzwerk notwendigen Spannung wird eine interne Energieversorgung, d.h. eine Energiequelle in Form eines Akkus verwendet.

Die DE 102 35 956 A1 offenbart eine Anordnung zur drahtlosen Übertragung von Stellsignalen an mehrere medizinische Geräte. In den medizinischen Geräten sind Empfänger vorgesehen, die von Stelleinrichtungen drahtlos übermittelte Stellsignale des Senders, empfangen. Die Stelleinrichtungen sind mit auswechselbaren Energiespeichern versehen, die an einer zentralen Aufladestelle ausgetauscht oder aufgeladen werden können.

Um zu vermeiden, dass bei mehreren Stelleinrichtungen desselben Typs eine falsche Stelleinrichtung am medizinischen Gerät angemeldet wird, weisen die einzelnen Stelleinrichtungen Kennungen auf. Diese Kennungen können im Rahmen eines Anmeldeverfahrens berührungslos mittels optischer oder induktiver Übertragung übertragen werden.

Die W02006/050410 offenbart eine Vorrichtung und ein Verfahren zur Steuerung von medizinischen Geräten durch eine kabellose Fernsteuerung. Die drahtlose Verbindung zwischen der Fernsteuerung und dem zu steuernden Gerät kann beispielsweise durch Bluetooth erfolgen. Das Aufladen des Akkus in der Fernsteuerung erfolgt durch elektromagnetische Induktion. Es können auch mehrere drahtlose Fernsteuerungen in der näheren Umgebung verwendet werden. Um zu verhindern, dass durch verschiedene Fernsteuerungen erzeugte Signale die falschen Geräte ansteuern, wird jedes Gerät mit einem ID-Code ausgerüstet, das von der Fernsteuerung erkannt wird.

Nachteilig ist, dass bspw. Bluetooth eine Netzwerkübertragung ist, und das Netzwerk aus Fernsteuerung und zu steuerndem Gerät ständig aufrechterhalten werden muss, auch wenn gerade keine Steuerbefehle ausgetauscht werden. Dadurch ergibt sich ein relativ hoher Stromverbrauch der Fernsteuerung. Die Fernsteuerung wird deshalb durch einen Akku versorgt, der nach (fast jedem) Gebrauch geladen werden muss, um die Funktionsfähigkeit sicher zu stellen.

In manchen Operationsräumen wird aber die Stromversorgung zentral abgeschaltet, wenn diese nicht benutzt werden, oder das zu steuernde Gerät, etwa ein Operationsmikroskop, wird nach dem Gebrauch in einem Abstellraum ohne Stromanschluss gelagert, so dass ein Laden in der Nähe des Geräts nicht möglich ist. Die ausreichende Ladung der Fernsteuerung über die gesamte Lagerdauer ist damit nicht in jedem Fall sichergestellt, wenn die Fernsteuerung in der Nähe des Geräts verbleiben soll.

Nach dem Einschalten der Fernsteuerung und des zu steuernden Geräts muss zunächst die Netzwerkverbindung aufgebaut werden. Dies kann aber nur erfolgen, wenn der Akku der Fernsteuerung eine ausreichende Ladung aufweist. Außerdem könnte es passieren, dass eine baugleiche Fernsteuerung aus einem Nachbarraum eine ungewollte Verbindung mit dem zu steuernden Gerät aufbaut, wenn keine Vorsichtsmaßnahmen ergriffen werden. Das Risiko ist besonders groß, wenn die eigentliche Fernsteuerung des Gerätes nicht ausreichend geladen ist.

### Zugrunde liegende Aufgabe

Der Erfindung liegt als erste Aufgabe zugrunde, ein kabelloses Fernsteuerungssystem für ein medizinisches Gerät und ein solches Gerät mit einer Fernsteuerung zur Verfügung zu stellen, bei dem auch ohne permanenten Anschluss an die Netzstromversorgung ein ausreichender Ladezustand des Akkus einer Fernbedienung aufrecht erhalten werden kann.

Eine zweite Aufgabe besteht darin, ein Verfahren zum Aufladen eines Akkus einer Fernsteuerung zur Verfügung zu stellen.

### Erfindungsgemäße Lösung

Die erste Aufgabe wird durch ein kabelloses Fernsteuerungssystem mit den Merkmalen des Anspruchs 1 und ein medizinisches Gerät mit den Merkmalen des Anspruchs 7 gelöst. Die zweite Aufgabe wird durch ein Verfahren zum Aufladen eines Akkus einer Fernsteuerung nach Anspruch 12 gelöst. Die abhängigen Ansprüche enthalten vorteilhafte Weiterbildungen der Erfindung.

Erfindungsgemäß besteht die Lösung der Aufgabe in einem kabellosen Fernsteuerungssystem für ein medizinisches Gerät. Das Fernsteuerungssystem umfasst wenigstens einen ersten Akku, wenigstens einen zweiten Akku, ein Steuermodul zum Anbringen oder zum Einbauen in das medizinische Gerät, wenigstens eine Fernsteuerbedieneinheit und eine Ladestation. Der erste Akku ist in der Fernsteuerbedieneinheit angeordnet, und der zweite Akku ist der Ladestation zu deren Versorgung mit elektrischer Energie zugeordnet. Der zweite Akku kann insbesondere in die Ladestation integriert sein. Das Steuermodul ist zum Ausgeben von Steuersignalen für das medizinische Gerät ausgebildet. Die kabellose Fernsteuerbedieneinheit kann bspw. als Handfernsteuerung, als Fußschaltpult, als Mundschalter, etc. ausgebildet sein.

Dadurch, dass das Fernsteuerungssystem kabellos ist, wird eine Stolpergefahr durch auf dem Boden liegende Kabel innerhalb des Operationssaals vermieden. Außerdem erlaubt die kabellose Ausführung eine einfachere Aufstellung und Handhabung der Geräte. Der Ladestation, die insbesondere in das zu steuernde Gerät integriert oder an dieses angebracht sein kann, ist ein Akku zugeordnet, damit sie, wenn sie vom Stromnetz getrennt wird, eine in der Ladestation befindliche Fernsteuerbedieneinheit weiter mit Energie versorgen kann. Dadurch kann eine ausreichende Ladung in der Fernsteuerbedieneinheit zuverlässig gewährleistet werden, auch wenn das Gerät über einen längeren Zeitraum von der Netzstromversorgung getrennt bleiben sollte.

In einer vorteilhaften Weiterbildung verwendet das kabellose Fernsteuerungssystem eine Übertragungstechnik, die auf Funksignalen zwischen der Fernsteuerungbedieneinheit und dem Steuermodul am oder im zu steuernden Gerät basiert und insbesondere als eine Bluetooth-Netzwerkverbindung ausgeführt sein kann. Die Bluetooth-Verbindung ermöglicht eine drahtlose Funkvernetzung im sog. 2,4-GHz-ISM-Band, welches eine lizenzfreie Übertragung für industrielle, wissenschaftliche und medizinische Zwecke erlaubt.

Der Akku, welcher der Ladestation zugeordnet ist, kann eine Kapazität besitzen, die mindestens genauso hoch ist wie die Kapazität des Akkus in der Fernsteuerbedieneinheit, vorzugsweise sogar höher. Dadurch kann ein vollständiges Entladen des zweiten Akkus durch das Aufladen des ersten Akkus über eine lange Zeitdauer vermieden werden.

Außerdem können die Ladestation und die Fernsteuerbedieneinheit des kabellosen Fernsteuerungssystems jeweils eine Induktionsspule umfassen. Die Spulen sind dann derart aufeinander abgestimmt, dass ein Laden des ersten Akkus über ein induktives Magnetfeld möglich ist. Da das induktive Laden keine Steckkontakte benötigt, können die Flächen der Ladestation und der Fernsteuerbedieneinheit glatt gehalten werden, was ein Sterilisieren vereinfacht.

In dem kabellosen Fernsteuerungssystem können den Spulen der Ladestation und der Fernsteuerbedieneinheit jeweils ein Modulator/Demodulator zum Modulieren des induktiven Magnetfeldes zugeordnet sein. Zusätzlich zum induktiven Laden kann dann durch die Modulation/Demodulation des induktiven Magnetfeldes eine Signalübertragung für die Anmeldeprozedur zwischen der Fernsteuerbedieneinheit und dem zu steuernden Gerät im Rahmen einer induktiven Kopplung erfolgen. Durch die kurze Reichweite der induktiven Kopplung wird verhindert, dass es beim Anmelden zu einer ungewollten Verbindung zwischen weiter entfernten Geräten, die nicht angemeldet werden sollen, kommt. Ein weiterer Vorteil der Modulatoren/Demodulatoren, welche den beiden Ladespulen zugeordnet sind, ist, dass zusätzlich zu den Spulen keine weitere induktive Kopplung für die induktive Anmeldeprozedur notwendig ist.

Erfindungsgemäß wird außerdem ein medizinisches Gerät, insbesondere ein Operationsmikroskop, mit einem erfindungsgemäßen kabellosen Fernsteuerungssystem zur Verfügung gestellt. Das medizinische Gerät kann aber auch ein anderes optisches Beobachtungsgerät, beispielsweise ein Endoskop, sein. Auch chirurgische Geräte, wie etwa Geräte zur intraoperativen Strahlentherapie kommen als Geräte in Frage, die durch das erfindungsgemäße Fernsteuerungssystem gesteuert werden können. Ein anderes in Frage kommendes chirurgisches Gerät ist ein Phaco-Gerät. Ein solche umfasst eine sog. Phaco-Spitze, auch Phaco-Endstück genannt, und findet zum Entfernen der Linse des Verwendung. Phaco-Geräte weisen beispielsweise einen Ultraschallgenerator auf, welcher die Linse zerkleinernde Ultraschallschwingungen erzeugt, die zum distalen Ende der Phaco-Spitze geleitet werden. Mit dem distalen Ende wird die Ultraschallschwingung gezielt in die Linse eingebracht, um diese zu zerkleinern. Durch eine Absaugleitung wird die zerkleinerte Linse dann abgesaugt. Daneben können Phaco-Spitzen auch Klingen aufweisen, mit denen sich Schnitte in der Linse setzen lassen.

Das Steuermodul und/oder die Ladestation können in das medizinische Gerät eingebaut sein. Dadurch werden zusätzliche Gehäuseteile vermieden und eine einfache und übersichtliche Gestaltung des medizinischen Geräts erreicht. Außerdem ist die Fernsteuerbedieneinheit dann immer beim Gerät, wenn sie geladen wird. Sie ist daher immer griffbereit, wenn das Gerät in Betrieb genommen werden soll. In diesem Fall ist auch der zweite Akku, also der der Ladeeinheit zugeordnete Akku, in das medizinische Gerät eingebaut.

Das Steuermodul und/oder insbesondere die Ladestation können aber auch am medizinischen Gerät lösbar befestigt sein. Die Ladestation ist dann mobil und unabhängig vom Aufstellungsort des medizinischen Gerätes einsetzbar. Der der Ladestation zugeordnete Akku ist in diesem Fall in die Ladestation integriert.

Weiterhin wird ein Verfahren zum Aufladen eines in einer Fernsteuerbedieneinheit eines erfindungsgemäßen Fernsteuerungssystems angeordneten Akkus zur Verfügung gestellt, in dem die zum Laden des ersten Akkus benötigte Energie vom zweiten Akku zur Verfügung gestellt wird. Dadurch ist ein Laden des ersten Akkus möglich, wenn das Ladegerät keine Verbindung mit einem Stromnetz aufweist.

Vorteilhafterweise wird der zweite Akku geladen, während das Fernsteuerungssystem in Betrieb ist. Dann ist der zweite Akku in der Regel voll aufgeladen, wenn das Fernsteuerungssystem außer Betrieb genommen und vom Stromnetz getrennt wird.

Die Signalübertragung von der Fernsteuerbedieneinheit zu dem zu steuernden Gerät kann mittels einer Funk-Netzwerkverbindung erfolgen. Vorteil einer Funk-Verbindung ist, wie oben erwähnt, eine Vernetzung von Geräten ohne störende Kabel.

Bei einer vorteilhaften Weiterbildung der Erfindung erkennt die Fernsteuerbedieneinheit, wenn sie keinen Ladekontakt zur Ladestation hat und die Funk-Netzverbindung zum Gerät über eine bestimmte Zeitdauer unterbrochen ist. Sie erzeugt dann ein Signal, bspw. ein Lichtsignal oder einen Signalton. Wenn die Fernsteuerbedieneinheit keine Netzwerkverbindung zum Gerät feststellt, kann dies bedeuten, dass das Gerät ausgeschaltet ist. Falls zudem kein Ladekontakt zur Ladestation besteht, besteht die Gefahr, dass das Gerät mit dem Fernsteuerungssystem ohne Stromversorgung gelagert ist und vergessen wurde, den Ladekontakt der Fernsteuerbedieneinheit zur Ladestation herzustellen. Durch das Signal kann das Bedienpersonal alarmiert werden, so dass es die Fernsteuerbedieneinheit in Ladekontakt bringen kann, um ein Aufladen ihres Akkus sicherzustellen.

Die Fernsteuerbedieneinheit kann außerdem ein Warnsignal erzeugen, wenn der Ladezustand ihres Akkus zu niedrig ist. Das Warnsignal kann akustisch oder optisch sein. Auf diese Weise kann das Bedienpersonal alarmiert werden, wenn ein Nachladen vorgenommen werden muss.

Zusätzlich kann die Fernsteuerbedieneinheit den Ladezustand ihres Akkus optisch anzeigen. Dadurch wird eine automatische Information des Bedienpersonals über den Ladezustand des Akkus in der Fernsteuerbedieneinheit zur Verfügung gestellt, so dass ein Laden der Fernsteuerbedieneinheit veranlasst werden kann, bevor eine Mindestladung ihres Akkus überschritten ist.

Erfindungsgemäß kann die Aufladung des ersten Akkus durch den zweiten Akku kontaktlos, bspw. induktiv erfolgen. Dadurch werden Ladekabel vermieden. Glatte und sterilisierbare Oberflächen von Fernsteuerbedieneinheit und Ladestation sind möglich. Es ist aber grundsätzlich auch ein Laden über Ladekontakte, bspw. in Form von Steckkontakten, möglich.

Die Anmeldung der Fernsteuerbedieneinheit am Steuermodul an oder in dem zu steuernden Gerät kann vorteilhafterweise ebenfalls über die induktive Kopplung der Ladestation erfolgen. Es werden dann keine weiteren zusätzlichen Komponenten benötigt.

### Kurzbeschreibung der Zeichnungen

Weitere Merkmale, Eigenschaften und Vorteile der Erfindung ergeben sich aus der nachfolgenden Beschreibung von Ausführungsbeispielen unter Bezugnahme auf die beiliegenden Figuren. Es zeigen:
- Fig. 1: ein Operationsmikroskop mit einem beweglichen Stativ.
- Fig. 2: die Freiheitsgrade des Stativs aus Fig. 1.
- Fig. 3: eine schematische Darstellung eines Fernsteuerungssystems in der Aufladephase des Akkus in dem zu steuernden Gerät.
- Fig. 4: ein stark vereinfachtes Blockschaltbild der Ladestation und der Fernsteuerbedieneinheit.
- Fig. 5: das Fernsteuerungssystem aus Fig.3 während des Ladens des Akkus in der Fernsteuerbedieneinheit.
- Fig. 6: eine alternative Anordnung der Elemente eines Fernsteuerungssystems beim Laden des Akkus in der Fernsteuerbedieneinheit.

### Detaillierte Beschreibung des Ausführungsbeispiels

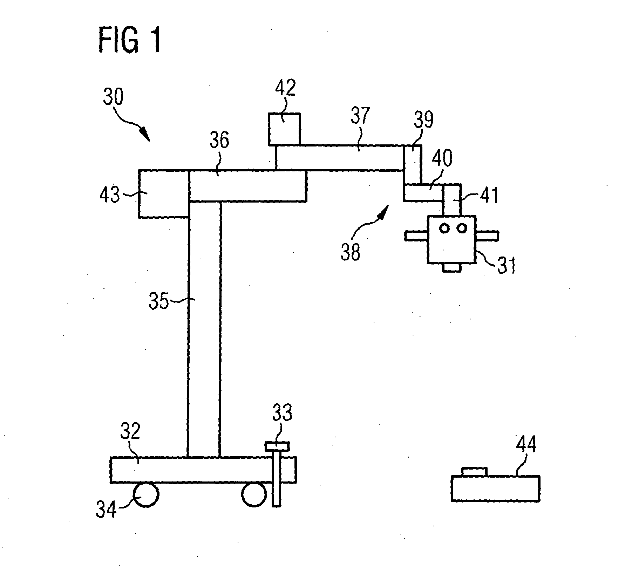

Fig. 1 zeigt schematisch ein Operationsmikroskop 31 mit einem Stativ 30 als ein Beispiel für ein medizinisches Gerät, bei dem ein erfindungsgemäßes Fernsteuerungssystem verwendet werden kann. Das Stativ 30 ruht auf einem Fuß 32, an dessen Unterseite Rollen 34 vorhanden sind, die ein Verfahren des Stativs 30 ermöglichen. Um ein ungewolltes Verfahren des Stativs 30 zu verhindern, besitzt der Fuß 32 außerdem eine Fußbremse 33.

Zum Fernsteuern des Stativs und/oder des Operationsmikroskops 31 ist ein Fußschaltpult 44 als Fernsteuerbedieneinheit vorhanden, das über eine Funk-Netzwerkverbindung mit einem Steuermodul (nicht dargestellt) im Operationsmikroskop 31 verbunden ist.

Das Stativ 30 umfasst eine höhenverstellbare Stativsäule 35, einen Tragarm 36, einen Federarm 37 und eine Mikroskopaufhängung 38, welche wiederum ein Verbindungselement 39, einen Schwenkarm 40 und einen Haltearm 41 umfasst.

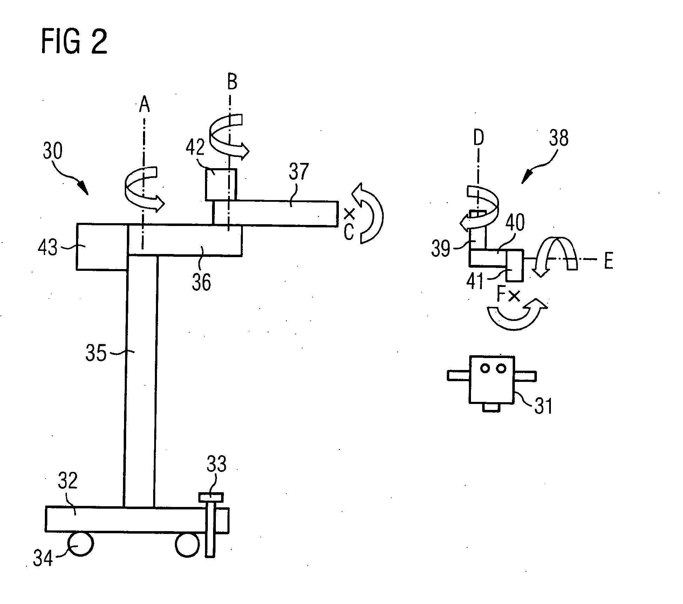

Die Freiheitsgrade, die die Stativglieder zum Positionieren des Operationsmikroskops 31 zur Verfügung stellen, sind in Fig. 2 dargestellt. Der Tragarm 36 ist an seinem einen Ende um eine Achse A drehbar mit der Stativsäule 35 verbunden. Am anderen Ende des Tragarms 36 ist ein Ende des Federarms 37 um eine zur Achse A parallele Achse B drehbar befestigt, so dass der Tragarm 36 und der Federarm 37 einen Gelenkarm bilden. Das andere Ende des Federarms 37 ist von einem Kippmechanismus gebildet (nicht dargestellt), an dem die Mikroskopaufhängung 38 befestigt ist und der ein Verkippen der Mikroskopaufhängung 38 um die Achse C ermöglicht.

Die Mikroskopaufhängung 38 weist eine Drehachse D, eine Schwenkachse E und eine Kippachse F auf, um die sich das Mikroskop 31 drehen, schwenken und kippen lässt. Mit einem Verbindungselement 39 ist die Mikroskopaufhängung 38 am äußeren Ende des Federarms 37 um die Drehachse D drehbar befestigt. Die Drehachse D erstreckt sich entlang des Verbindungselementes 39. An das Verbindungselement 39 schließt sich ein Schwenkarm 40 an, mit dessen Hilfe sich das Mikroskop 31, genauer gesagt, ein am Schwenkarm 40 angebrachter Haltearm 41, an dem das Mikroskop 31 mittels einer Mikroskophalterung (nicht dargestellt) befestigt ist, um die Schwenkachse E schwenken lässt. Die Schwenkachse E erstreckt sich durch den Schwenkarm 40. Der Winkel zwischen Schwenkarm 40 und Verbindungselement 39, d.h. der Winkel zwischen der Schwenkachse E und der Drehachse D, kann mittels einem zwischen dem Verbindungsteil 39 und dem Schwenkarm 40 angeordneten Verstellmechanismus variiert werden.

Durch den Haltearm 41 verläuft senkrecht zur Darstellungsebene die Kippachse F, die ein Kippen des Operationsmikroskops 31 ermöglicht. Das Operationsmikroskop 31 ist mittels einer nicht dargestellten Mikroskophalterung am Haltearm 41 befestigt.

Um ein ungewolltes Verstellen des Mikroskops 31 aus einer gewählten Position zu verhindern, sind die Stativglieder bzw. die Gelenke zwischen den Stativgliedern mit Bremsen (nicht dargestellt) versehen, welche nach dem Positionieren des Mikroskops 31 fixiert werden. Als Bremsen kommen sowohl manuell als auch elektrisch zu betätigende Bremsen in Frage.

Am Stativ 30 sind außerdem eine Lichtquelle 42 zur Objektbeleuchtung sowie ein Netzanschlussgerät und ein Bedienelement 43 für elektrische Komponenten des Mikroskops 31 und ggf. des Stativs 30 angeordnet.

Mittels des Fußschaltpultes 44 werden die optischen Einstellungen und die Feinpositionierung des Mikroskops vorgenommen. Die übrigen Einstellmöglichkeiten, insbesondere die oben beschriebenen Einstell- und Fixiermöglichkeiten, können über weitere Fernsteuerbedieneinheit betätigt werden.

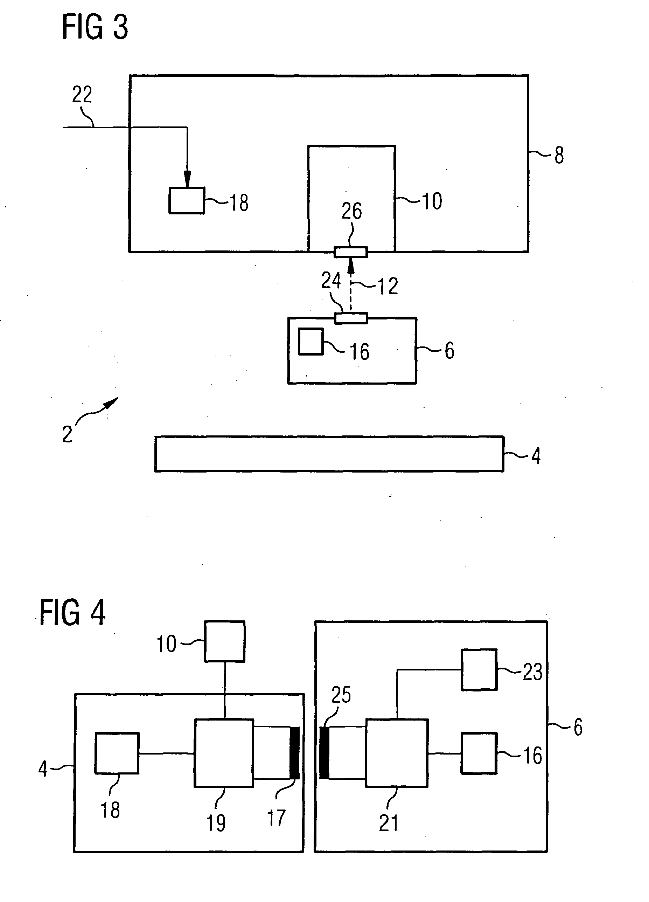

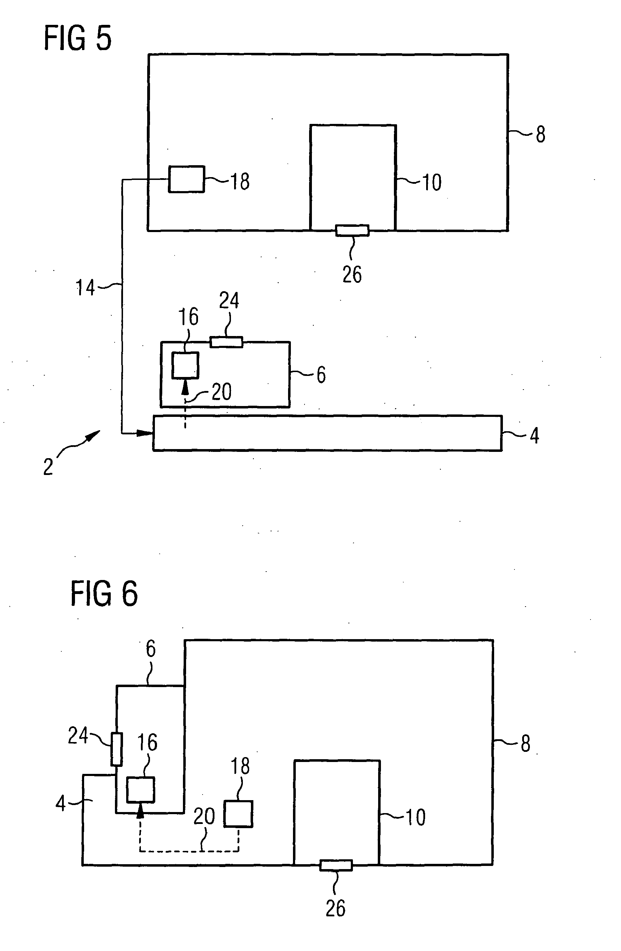

Die Fig. 3 und 5 zeigen ein erstes Ausführungsbeispiel für ein erfindungsgemäßes Fernsteuerungssystem 2. Dieses umfasst eine Ladestation 4, eine Fernsteuerbedieneinheit 6, die nachfolgend kurz Fernsteuerung genannt wird, und eine Steuereinrichtung 10, die in ein Operationsmikroskop 31 als zu steuerndem Gerät 8 integriert ist. Die Fernsteuerung 6 weist einen ersten Akku 16, im Folgenden auch Fernsteuerungsakku 16 genannt, und einen Sender 24 auf. Das zu steuernden Gerät 8 ist mit einem zweiten Akku 18, im Folgenden auch Geräteakku 18 genannt, der der Ladestation 4 zugeordnet ist, sowie mit einem mit der Steuereinrichtung 10 verbundenen Empfänger 26 ausgerüstet. Der Geräteakku 18 dient der Ladestation 4 als Stromquelle falls sie nicht an ein Stromnetz angeschlossen ist. Da die Ladestation 4 im vorliegenden Ausführungsbeispiel nicht in das zu steuernde Gerät 8 integriert ist, sondern als individuelle Einheit ausgebildet ist, ist sie über eine leitende Verbindung, die beispielsweise als Kabel ausgebildet sein kann, an den Geräteakku 18 anschließbar. Die leitende Verbindung kann aber auch als eine Steckverbindung zwischen der Ladestation 4 und der Außenseite des zu steuernden Geräts 8 ausgebildet sein. Der Fernsteuerungsakku 16 ist in der Fernsteuerung 6 angeordnet (Fig. 3 und 5).

Der Sender 24 der kabellosen Netzwerkverbindung ist ein Funksender, bspw. ein Bluetooth-Sender, der Empfänger 26 eine Funkempfänger, beispielsweise ein Bluotooth-Empfänger.

In einer alternativen Anordnung des erfindungsgemäßen Fernsteuerungssystems kann auch die Ladeeinheit 4 in das medizinische Gerät 8 integriert sein. Ebenso kann auch der zweite Akku 18 in das medizinische Gerät 8 eingebaut sein.

Fig. 3 stellt das Fernsteuerungssystem 2 während der Aufladephase des Akkus 18, der im vorliegenden Ausführungsbeispiel in das zu steuernde Gerät 8 integriert ist, dar. Der Geräteakku 18 wird durch einen Netzanschluss 22 aufgeladen, während das zu steuernde Gerät 8 in Betrieb ist. Während des Betriebs versorgt der vorher aufgeladene Fernsteuerungsakku 16 die Fernsteuerung 6 mit Energie.

Im Betrieb des Fernsteuerungssystems 2 sendet der Sender 24 der Fernsteuerung 6 Steuersignale 12 über eine drahtlose Netzwerkverbindung, etwa Bluetooth oder W-LAN, an den Empfänger 26 der Steuereinheit 10. Die Signale werden in der Steuereinheit 10 verarbeitet und in elektrische Signale zum Einwirken auf die Stellglieder des zu steuernden Gerätes 8, hier des Operationsmikroskops 31 oder auch des Stativs 30, umgewandelt.

Das Laden des Fernsteuerungsakkus 16 erfolgt durch den Geräteakku 18 mittels induktiver Kopplung, wenn sich die Fernsteuerung 6 in der Ladestation 4 befindet. In Fig. 4 ist die induktive Kopplung zwischen der Fernsteuerung 6 und der Ladestation 4 in Form eines stark vereinfachten Blockschaltbildes dargestellt.

Die Fernsteuerung 6 umfasst einen Prozessor 23, einen Modulator 21, der auch als Demodulator arbeiten kann, und eine Spule 25. Eine zweite Spule 17 und ein zweiter Modulator 19, der ebenfalls als Demodulator arbeiten kann, sind in der Ladestation 4 angeordnet.

In der Fernsteuerung 6 ist der Prozessor 23 mit dem ersten Modulator/Demodulator 21 verbunden, der wiederum mit der ersten Spule 25 verbunden ist. Der Modulator/Demodulator 19 in der Ladestation 4 ist sowohl an die Spule 17 als auch an die im zu steuernden Gerät 8 eingebaute Steuereinheit 10 angeschlossen, beispielsweise über ein weiteres Kabel oder, falls eine Steckverbindung zwischen Ladestation und Gerät 8 vorhanden ist, über die Steckverbindung.

Über die induktive Kopplung erfolgt außer dem Laden des Fernsteuerungsakkus 16 auch die Anmeldung der Fernsteuerung 6 bei der Steuereinheit 10.

Wenn die Fernsteuerung 6 an das Gerät 8 angemeldet wird, gibt der Prozessor 23 die digitalen Signale der Anmeldeprozedur an den ersten Modulator 21 aus. Dieser moduliert auf der Basis der digitalen Signale das von der ersten Spule 25 erzeugte induktive Magnetfeld. Dieses modulierte Magnetfeld induziert dann in der zweiten Spule 17 eine modulierte Spannung, die von dem Demodulator 19 demoduliert und in digitale Signale umgewandelt wird. Die digitalen Signale werden einem Prozessor (nicht dargestellt) der Steuereinrichtung 10 zugeführt. Der beschrieben Ablauf kann im Rahmen der Anmeldeprozedur auch in umgekehrter Richtung ablaufen.

Es können mehrere Fernsteuerungen 6 des gleichen Typs vorhanden sein. Damit die verschiedenen Fernsteuerungen die richtigen Geräte ansteuern, muss jede Fernbedienung an das entsprechende Gerät angemeldet werden. Die Anmeldung der jeweiligen Fernsteuerung 16 an das jeweilige Gerät 8 erfolgt über die induktive Kopplung, die oben beschrieben ist. Aufgrund der kurzen Reichweite der induktiven Kopplung erfolgt die Anmeldung in der Regel am nächstgelegenen Gerät. Da sich die Fernbedienung üblicherweise im selben Raum wie das Gerät befindet und die induktive Kopplung normalerweise nur innerhalb des Raumes durchführbar ist, kann ein Anmelden an einem "falschen" Gerät im Nachbarraum, das über die drahtlose Netzwerkverbindung problemlos erreichbar wäre, zuverlässig vermieden werden.

Fig. 5 zeigt das Fernsteuerungssystem 2 aus Fig. 3 während der Aufladephase des Akkus 16 in der Fernsteuerung 6. Der aufgeladene Geräteakku 18 in dem zu steuernden Gerät 8 ist über die leitende Verbindung 14 mit der Ladestation 4 verbunden und versorgt diese mit Energie. Die Fernsteuerung 6 mit dem Fernsteuerungsakku 16 befindet sich an der Ladestation 4, die den Fernsteuerungsakku 16 durch induktive Kopplung kontaktlos auflädt (s. Fig. 4). Beim Laden wird der Strom von der Spule 25 um den Demodulator 21 herumgeführt (im Blockschaltbild nicht dargestellt) und direkt in den Akku 16 geleitet. Da das induktive Laden kontaktlos funktioniert, braucht sich die Fernsteuerung 6 beim Laden nur in der Nähe der Ladestation zu befinden. Um eine optimale Induktion in der Spule 25 der Fernsteuerung 10 zu gewährleisten, kann es jedoch vorteilhaft sein, die Ladestation 4 so auszubilden, dass sie ein Fixieren der Fernsteuerung 6 in einer bestimmten Position relativ zur Ladestation ermöglicht.

Wenn der Geräteakku 18 eine größere Kapazität hat als der Fernsteuerungsakku 16, kann er über einen längeren Zeitraum Ladeenergie für den Fernsteuerungsakku 16 zur Verfügung stellen. Das Gleiche lässt sich erreichen, wenn statt eines Geräteakkus mit höherer Kapazität als die des Fernsteuerungsakkus zwei oder mehr Geräteakkus 18 mit derselben Kapazität wie die des Fernsteuerungsakkus 16 vorhanden sind.

In Fig. 6 ist ein zweites Ausführungsbeispiel für das erfindungsgemäße Fernsteuerungssystem dargestellt. In diesem Ausführungsbeispiel ist die Ladestation 4 in das zu steuernde Gerät 8 integriert und dient gleichzeitig als Halterung für die Fernsteuerung 6. Der Geräteakku 18 kann in die Steuereinrichtung 10 oder in die Ladestation 4 integriert sein. Im Übrigen unterscheidet sich das zweite Ausführungsbeispiel nicht vom ersten Ausführungsbeispiel.

Gleiche Elemente sind daher in beiden Ausführungsbeispielen mit denselben Bezugszeichen versehen.

Alle einstellbaren Elemente des Stativs 30 und die optische Einstellungen des Mikroskops können mit Hilfe des erfindungsgemäßen Fernsteuerungssystems durch elektrische Stelleinrichtungen eingestellt werden. Als Fernbedienung ist statt des in den Zeichnungen dargestellten Fußschaltpults 44 auch eine manuell bedienbare oder mit dem Mund zu bedienende Fernsteuerung möglich.

Obwohl in den Ausführungsbeispielen jeweils nur ein Akku in der Fernsteuerung 6 beschrieben worden ist, kann die Fernsteuerung 6 auch zwei oder mehr Akkus 16 umfassen.

### Bezugszeichenliste

- 2: Fernsteuerungssystem
- 4: Ladestation
- 6: Fernsteuerung
- 8: zu steuerndes Gerät
- 10: Steuereinrichtung
- 12: Steuersignal
- 14: leitende Verbindung
- 16: Fernsteuerungsakku
- 17: Erste Spule
- 18: Geräteakku
- 19: Erster Modulator/Demodulator
- 20: Induktive Kopplung
- 21: Zweiter Modulator/Demodulator
- 22: Netzanschluss
- 23: Prozessor der Fernsteuerung
- 24: Sender
- 25: Zweite Spule
- 26: Empfänger
- 30: Stativ
- 31: Mikroskop
- 32: Fuß
- 33: Fußbremse
- 34: Rollen
- 35: Stativsäule
- 36: Tragarm
- 37: Federarm
- 38: Aufhängung
- 39: Verbindungselement
- 40: Schwenkarm
- 41: Haltearm
- 42: Lichtquelle
- 43: Bedienelement
- 44: Fußschaltpult

## Patentansprüche

1. Kabelloses Fernsteuerungssystem (2) für ein medizinisches Gerät (8), das wenigstens einen ersten Akku (16), wenigstens einen zweiten Akku (18), ein Steuermodul (10) zum Anbringen oder zum Einbauen in das medizinische Gerät (8), wenigstens eine Fernsteuerbedieneinheit (6) und eine Ladestation (4) umfasst, wobei der erste Akku (16) in der Fernsteuerbedieneinheit (6) angeordnet ist und der zweite Akku (18) der Ladestation (4) zugeordnet ist.

2. Kabelloses Fernsteuerungssystem (2) nach Anspruch 1, in dem die Verbindung zwischen der Fernsteuerbedieneinheit (6) und dem zu steuernden Gerät (8) eine Funk-Netzwerkverbindung umfasst.

3. Kabelloses Fernsteuerungssystem (2) nach Anspruch 1 oder 2, in dem der zweite Akku (18) eine Kapazität besitzt, die höher ist, als die des ersten Akkus (16).

4. Kabelloses Fernsteuerungssystem (2) nach einem der Ansprüche 1 bis 3, in dem die Ladestation (4) und die Fernsteuerbedieneinheit (6) jeweils eine Spule (17, 25) umfassen und die Spulen (17, 25) derart aufeinander abgestimmt sind, dass ein Laden des ersten Akkus (16) über ein induktives Magnetfeld möglich ist.

5. Kabelloses Fernsteuerungssystem (2) nach Anspruch 3, in dem der Spule (17) der Ladestation (4) ein Modulator/Demodulator zum Modulieren und Demodulieren des induktiven Magnetfeldes zugeordnet ist und der Spule (25) der Fernsteuerbedieneinheit (6) ein Modulator/Demodulator zum Modulieren und Demodulieren des induktiven Magnetfeldes zugeordnet ist.

6. Kabelloses Fernsteuerungssystem (2) nach einem der vorhergehenden Ansprüche, in dem der zweite Akku (18) in die Ladestation (4) integriert ist.

7. Medizinisches Gerät (8) mit einem Fernsteuerungssystem (2) nach einem der vorhergehenden Ansprüche.

8. Medizinisches Gerät (8) nach Anspruch 7, in dem das Steuermodul (10) in das medizinische Gerät (8) eingebaut ist.

9. Medizinisches Gerät (8) nach Anspruch 7 oder 8, in dem die Ladestation (4) in das medizinische Gerät (8) eingebaut ist.

10. Medizinisches Gerät (8) nach Anspruch 9, in dem der zweite Akku (18) in das medizinische Gerät eingebaut ist.

11. Medizinisches Gerät (8) nach Anspruch 7 oder 8, in dem die Ladeeinheit (4) am medizinischen Gerät (8) lösbar befestigt ist.

12. Verfahren zum Aufladen eines in einer Fernsteuerbedieneinheit (6) eines Fernsteuerungssystems nach einem der Ansprüche 1 bis 6 angeordneten Akkus (16), in dem die zum Laden des ersten Akkus (16) benötigte Energie vom zweiten Akku (18) zur Verfügung gestellt wird.

13. Verfahren nach Anspruch 12, in dem der zweite Akku (18) geladen wird, während das Fernsteuerungssystem in Betrieb ist.

14. Verfahren nach Anspruch 12 oder Anspruch 13, in dem die Signalübertragung von der Fernsteuerung zu dem zu steuernden Gerät mittels einer Funk-Netzwerkverbindung erfolgt.

15. Verfahren nach Anspruch 14, in dem die Fernsteuerbedieneinheit (6) ein Signal ausgibt, wenn sie keinen Ladekontakt zur Ladestation (4) hat und die Funk-Netzwerkverbindung über eine bestimmte Zeitdauer unterbrochen ist.

16. Verfahren nach einem der Ansprüche 12 bis 15, in dem das Aufladen des ersten Akkus (16) durch den zweiten Akku (18) kontaktlos erfolgt.

17. Verfahren nach Anspruch 16, in dem in dem das Aufladen des ersten Akkus (16) durch den zweiten Akku (18) induktiv erfolgt.

18. Verfahren nach Anspruch 17, in dem eine Anmeldung der Fernsteuerbedieneinheit (6) am Steuermodul (10) über die induktive Kopplung der Ladestation (4) erfolgt.

19. Verfahren nach einem der Ansprüche 12 bis 18, in dem die Fernsteuerbedieneinheit (6) den Ladezustand des ersten Akkus (16) anzeigt

20. Verfahren nach einem der Ansprüche 12 bis 19, in dem die Fernsteuerbedieneinheit (6) ein Warnsignal erzeugt, wenn der Ladezustand des ersten Akkus (16) zu niedrig ist.
